(19) Europäisches Patentamt
European Patent Office
Office européen des brevets

(11) Publication number: 0 273 744 B1

(12) **EUROPEAN PATENT SPECIFICATION**

(45) Date of publication of the patent specification: **01.08.90**

(51) Int. Cl.⁵: **A61K 31/42,** C07D 261/12, C07D 261/20

(21) Application number: **87311455.7**

(22) Date of filing: **24.12.87**

(54) **Centrally-acting muscle relaxants.**

(30) Priority: **26.12.86 JP 312843/86**

(43) Date of publication of application: **06.07.88 Bulletin 88/27**

(45) Publication of the grant of the patent: **01.08.90 Bulletin 90/31**

(84) Designated Contracting States: **AT BE CH DE ES FR GB GR IT LI LU NL SE**

(56) References cited:
**PATENT ABSTRACTS OF JAPAN, vol. 5, no. 98 (C-58)[761], 10th June 1981; & JP - A - 56 34674 (SANKYO K.K.) 06-04-1981**
**PATENT ABSTRACTS OF JAPAN, vol. 4, no. 159 (C-30)[641]; 6th November 1980; & JP - A - 55 104 274 (SANKYO K.K.) 09-08-1980**
**PATENT ABSTRACTS OF JAPAN, vol. 4, no. 129 (C-24)[611]; 10th September 1980; & JP - A - 55 83766 (SANKYO K.K.) 24-06-1980**
**PATENT ABSTRACTS OF JAPAN, vol. 3, no. 95 (C-55), 11th August 1979; & JP - A - 54 73775 (SANKYO K.K.) 13-06-1979**

(73) Proprietor: **Sankyo Company Limited, 5-1 Nihonbashi Honcho 3-chome Chuo-ku, Tokyo(JP)**

(72) Inventor: **Iwata,Nobuyoshi, Sankyo Company Limited No. 2-58 1-chome, Hiromachi Shinagawa-ku Tokyo 140(JP)**
Inventor: **Tonohiro, Toshiyuki, Sankyo Company Limited No. 2-58 1-chome, Hiromachi Shinagawa-ku Tokyo 140(JP)**
Inventor: **Hara, Takao, Sankyo Company Limited No. 2-58 1-chome, Hiromachi Shinagawa-ku Tokyo 140(JP)**
Inventor: **Nagano, Mitsuo, Sankyo Company Limited No. 2-58 1-chome, Hiromachi Shinagawa-ku Tokyo 140(JP)**

(74) Representative: **Gibson, Christian John Robert et al, MARKS & CLERK 57/60 Lincoln's Inn Fields, London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to centrally-acting muscle relaxants.

Myotony or spasms are often observed as the sequelae of cerebrovascular disorders such as cerebral apoplexy, or as cephalotraumatic sequelae, and make rehabilitation difficult. Centrally-acting muscle relaxants are available which can relieve such spasms, and act on the central nervous system. Examples of existing centrally-acting muscle relaxants include eperisone hydrochloride and afloqualone.

An object of the present invention is to provide new centrally-acting muscle relaxants. Such relaxants are needed which can alleviate myotony and spasms, preferably without giving rise to accompanying drowsiness.

It has now been discovered that some isoxazolin-3-one derivatives possess strong centrally-acting muscle relaxant activity.

The isoxazolin-3-one derivatives of this invention are represented by the general formula

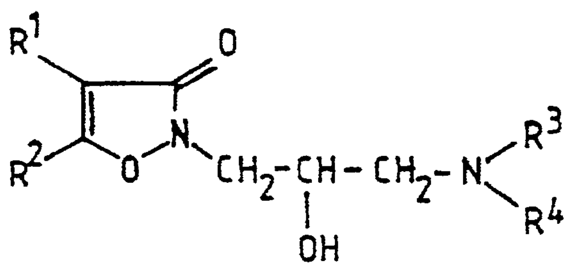

(I)

wherein:

for $R^1$ and $R^2$
<u>either</u>
$R^1$ represents a hydrogen atom or a halogen atom; and
$R^2$ represents an alkyl group with 1 to 4 carbon atoms, a phenyl group, a substituted phenyl group, a heterocyclic group, or a substituted heterocyclic group;

<u>or</u>
$R^1$ and $R^2$ together with the intervening carbon atoms form a 6- or 7-membered hydrocarbon ring;

for $R^3$ and $R^4$
<u>either</u>
$R^3$ represents a hydrogen atom or an alkyl group having from 1 to 4 carbon atoms; and $R^4$ represents an alkyl group having 1 to 4 carbon atoms;

<u>or</u>
$R^3$ and $R^4$ together with the intervening nitrogen atom form a 5- or 6-membered alicyclic amino group optionally having at least one further heteroatom selected from the group consisting of oxygen, sulphur and nitrogen heteroatoms. The isoxazolin-3-ones of formula (I) can be employed as their pharmaceutically acceptable salts.

The isoxazolin-3-ones of formula (I) are known from Japanese Patent, Laid Open (Kokai), 56-34674, and are said to have anti-inflammatory, analgesic and antipyretic activity.

The present invention resides in the unexpected and unpredictable discovery of centrally-acting muscle relaxant activity in the compounds of formula (I). There is no correlation between, on the one hand, an activity as an anti-inflammatory, analgesic and antipyretic agent, and, on the other hand, an activity as a centrally-acting muscle relaxant agent.

Thus, the present invention further resides in a method of effecting centrally-acting muscle relaxant activity, which comprises administering a compound of the formula (I) or a pharmaceutically acceptable salt thereof.

The present invention further resides in a centrally-acting muscle relaxant composition comprising a compound of formula (I) and a pharmaceutically acceptable carrier. Such compositions may be provided together with instructions for use in treating spasms, myotony or other symptoms, based on the activity of the compounds as centrally-acting muscle relaxants, and may be packaged.

<u>Preferred Embodiments of the Present Invention</u>

In the formula (I), $R^1$ can be a hydrogen atom, or a halogen atom, such as a fluorine, chlorine, or bromine atom.

$R^2$, $R^3$ and/or $R^4$ can be a straight or branched chain alkyl group with 1 to 4 carbon atoms, such as a methyl, ethyl, propyl, isopropyl, butyl, isobutyl or tert-butyl group.

When $R^2$ is a substituted phenyl group, it has 1 to 3 substituents selected from alkyl groups with 1 to 4 carbon atoms, such as a methyl, ethyl, propyl, isopropyl, butyl, isobutyl or tert-butyl group; alkoxy groups with 1 to 4 carbon atoms, such as a methoxy, ethoxy, propoxy, sopropoxy, butoxy, isobutoxy or tert-butoxy group; hydroxy groups; halogen atoms, such as a fluorine, chlorine, or bromine atom; nitro groups; and/or trifluoromethyl groups

When $R^2$ is a heterocyclic group, which is unsubstituted or substitute, it has 5 or 6 ring atoms which include 1 to 3 heteroatoms selected from oxygen, sulphur, and/or nitrogen heteroatoms. Examples of such heterocyclic groups include a furyl, thienyl, thiazolyl or pyridyl group.

When $R^2$ is a substituted heterocyclic group, it has 1 to 3 substituents selected from the substituents listed for the substituted phenyl groups.

$R^1$ and $R^2$, together with the intervening carbon atoms, can form a 6- or 7-membered hydrocarbon ring which is saturated, unsaturated or aromatic, and is unsubstituted. Examples of such rings include a benzene, cyclohexene or cycloheptene ring

When $R^3$ and $R^4$, together with the intervening nitrogen atom, form a 5- or 6-membered alicyclic amino group, the alicyclic ring can optionally have at least one further heteroatom selected from the group consisting of oxygen, sulphur and nitrogen heteroatoms. Furthermore, the nitrogen heteroatom in such an alicyclic ring can be substituted with an alkyl group having 1 to 4 carbon atoms. Examples of the alicyclic ring include a morpholino, 1-piperazinyl, 4-methyl-1-piperazinyl, 1-pyrrolidinyl or piperidino group.

Preferred compounds of formula (I) of this invention include the compounds wherein $R^1$ represents a hydrogen atom or a halogen atom; $R^2$ represents an alkyl group, a phenyl group in which the aromatic ring may be optionally substituted, or $R^1$ and $R^2$ form a benzene ring; $R^3$ represents a hydrogen atom and $R^4$ represents an alkyl group; or $R^3$ and $R^4$ form a 5-membered alicyclic amino group, or a 6-membered alicyclic amino group having at least one further heteroatom which is an oxygen heteroatom, a nitrogen heteroatom and/or a nitrogen heteroatom substituted with an alkyl group.

Especially preferred compounds of formula(I) of this invention include the compounds wherein $R^1$ represents a hydrogen atom or a fluorine, chlorine or bromine atom; $R^2$ represents an alkyl group, a phenyl group in which the aromatic ring may be optionally substituted with a fluorine atom, chlorine atom, bromine atom, alkoxy group, or hydroxy group; $R^3$ represents a hydrogen atom and $R^4$ represents an alkyl group; or $R^3$ and $R^4$ form a morpholino, 1-piperazinyl, 4-methyl-1-piperazinyl, or piperidino group.

Examples of the preferred compounds of this invention include the following compounds:

Compound 1

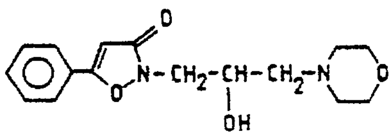

2-(2-hydroxy-3-morpholinopropyl)-5-phenyl-4-isoxazolin-3-one

Compound 2

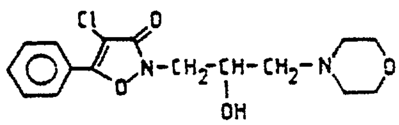

4-chloro-2-(2-hydroxy-3-morpholinopropyl)-5-phenyl-4-isoxazolin-3-one

Compound 3

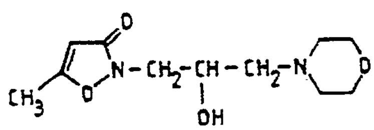

2-(2-hydroxy-3-morpholinopropyl)-5-methyl-4-isoxazolin-3-one

Compound 4

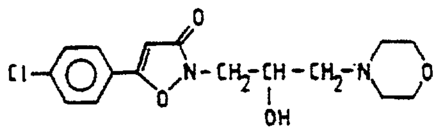

5-p-chlorophenyl-2-(2-hydroxy-3-morpholinopropyl)-4-isoxazolin-3-one

Compound 5

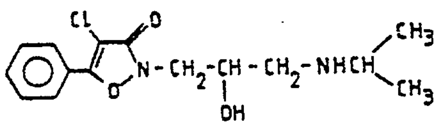

4-chloro-2-(2-hydroxy-3-isopropylaminopropyl)-5-phenyl-4-isoxazolin-3-one

Compound 6

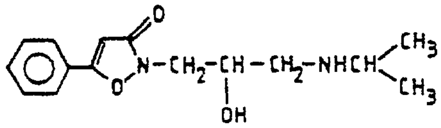

2-(2-hydroxy-3-isopropylaminopropyl)-5-phenyl-4-isoxazolin-3-one

Compound 7

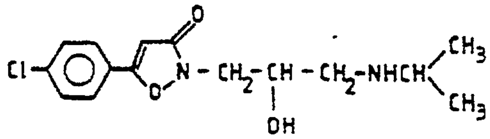

5-p-chlorophenyl-2-(2-hydroxy-3-isopropylaminopropyl)-4-isoxazolin-3-one

In the present invention, the isoxazolin-3-one derivatives of formula (I) can be employed as pharmaceutically acceptable acid addition salts. The salts can be exemplified by inorganic acid salts such as a hydrochloride, hydrobromide or sulfate salt; and organic acid salts such as an oxalate, lactate, citrate, tartarate, succinate, maleate, fumarate or methanesulfonate salt.

Asymmetric carbon atoms exist in the compounds represented by the general formula (I), and the present invention embraces the use of optical isomers and any mixtures thereof.

Isoxazolin-3-one derivatives having the general formula (I) can be prepared according to the procedures described in the Japanese Patent, Laid Open (Kokai), 56-34674, to which the reader is referred, and incorporated herein by reference.

Specifically, a 2-(3-halo-2-hydroxypropyl-4-isoxazolin-3-one can be reacted with an amine of formula $NHR^3R^4$, in accordance with the following reaction scheme:

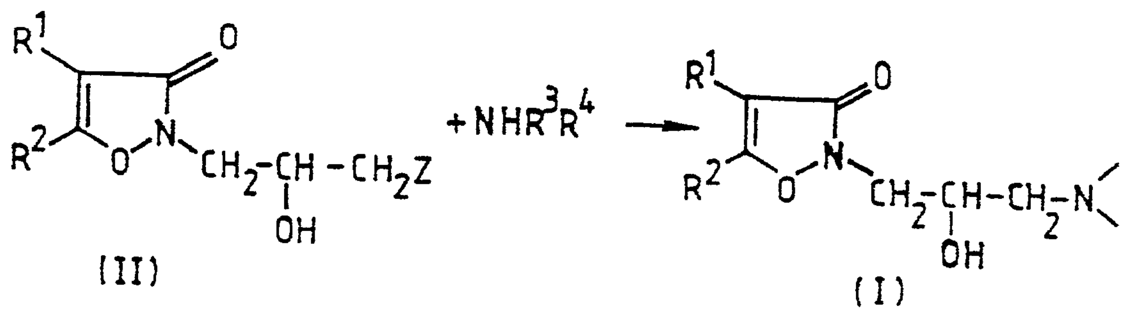

(where $R^1$, $R^2$, $R^3$ and $R^4$ are as defined, and Z represents a halogen atom).

For example, the isoxazolin-3-one of formula (II) can be reacted with the amine $NHR^3R^4$ in a solvent at reflux for several hours, and the product then isolated using conventional techniques. Further details can be found in the Japanese Kokai.

In general, the compounds of formula (I) do not induce much drowsiness, have low toxicity and have a centrally-acting muscle relaxant activity. Typically the compounds are rapidly absorbed after oral administration, intraduodenal or intraperitoneal administration and manifest the muscle relaxant effect. They are expected to be particularly useful as centrally acting muscle relaxants for cerebral apoplectic sequelae and cephalo-traumatic sequelae, and for spastic spinal paralysis, cervicospinal disease post-operative sequelae (including tumor of the brain and spinal cord), traumatic sequelae (spinal cord injury, cephalic trauma), amyotrophic lateral sclerosis, cerebral palsy, spinocerebellar degeneration, disorders of the spinal cord vessel, SMON (sub-acute myelo-optic neuropathy), caisson disease, spasmic paralysis due to any other spinal cord disease, and increased myotony such as systemic cramp or shoulder stiffness.

Examples of modes of administration include oral administration by tablets, capsules, granules, powders and syrups, and parenteral administration by injections and suppositories. These various kinds of compositions can be made according to the usual procedures where the main ingredient is mixed with suitable additives, such as carriers, vehicles, binders, disintegrators, lubricants, corrigents, etc, as occasion demands. Though the dosage may be variable depending on the symptom, age, body weight, etc, of the patient, in general a dose of 5 mg to 50 mg may be given orally to adult humans, 1 to 3 times a day.

Test Results

The following test results of pharmacological and toxicity studies demonstrate that the compounds of formula (I) of this invention have utility as centrally-acting muscle relaxants with low toxicity.

Test 1: Suppression of decerebrate rigidity (rats)

The lowering of decerebrate rigidity in laboratory animals such as rats or cats is a well known model for control of human spasticity and/or rigidity.

In the test method, a rat was anesthetized with Halothane (Trade Mark) and fixed on a stereotaxic apparatus. In accordance with the brain map by Pellegrino et al., (L J Pellegrino, A S Pellegrino ? A. J. Cushman : A Stereotaxic Atlas of the Rat Brain, Plenum Press, New York and London (1967)), an electrode 0.7 mm in diameter which was insulated except for 1 mm at the tip was inserted bilaterally into the midbrain reticular formation (AP: 0, L: ±1.5, H: -3.0). Using the electrode, a high frequency electric current (100 KHz, 10 to 20 mA) was applied from a lesion generator (Glass Co., LM4A) for 2 to 3 minutes to cauterize electrically this brain region.

A clip serving as the reference electrode was fixed on the scalp intima. The rat was then immediately set free from the stereotaxic apparatus. A polyethylene cannula was inserted into the duodenum and

fixed with a bonding agent. After completion of these operations, Halothane anesthesia was immediately discontinued.

After 1.5 hours when the animal awoke from anesthesia, the rat was fixed on apparatus devised in our laboratory for a hind leg fixation. Both hind legs were fixed at the anterior part of the ankle joints. Both hindlimb paws were then pushed for 6 seconds per minute. The resulting repulsion was drawn on a polygraph through a strain gauge.

Test compounds were suspended in 0.5% CMC solution, and administered intraduodenally or orally by using a cannula inserted previously, or adminstered intraperitoneally.

The results are summarized in the following table, where the Compounds 1 to 7 have the structures given above, Compound 8 is the known compound eperisone hydrochloride, and Compound 9 is the known compound afloqualone.

Inhibition of decerebrate rigidity in rats

| Compound | Dose (mg/kg) (Administration route) | Onset of the effect (minutes) | Maximum inhibition (%) | Duration of the effect (minutes) |
|---|---|---|---|---|
| 1 | 10 (i.d.) | 10 | 20 | >90 |
| 1 | 30 (i.d.) | 5 | 60 | >180 |
| 1 | 30 (p.o.) | 10 | 95 | >160 |
| 1 | 30 (i.p.) | 5 | 60 | >120 |
| 2 | 50 (i.p.) | 10 | 50 | >120 |
| 2 | 30 (p.o.) | 5 | 55 | >90 |
| 2 | 50 (p.o.) | 5 | 90 | >90 |
| 2 | 100 (p.o.) | 5 | 100 | >90 |
| 3 | 50 (i.p.) | 5 | 30 | 50 |
| 4 | 50 (i.p.) | 5 | 80 | >120 |
| 5 | 50 (i.p.) | 5 | 95 | >120 |
| 6 | 50 (i.p.) | 5 | 30 | 60 |
| 7 | 50 (i.p.) | 5 | 75 | 60 |
| 8 | 10 (p.o.) | 10 | 30 | >30 |
| 8 | 30 (p.o.) | 10 | 30 | 45 |
| 8 | 30 (i.p.) | 5 | 80 | >180 |
| 8 | 50 (p.o.) | 10 | 50 | 30 |
| 8 | 100 (p.o.) | 5 | 50 | 60 |
| 9 | 5 (p.o.) | 10 | 25 | 60 |
| 9 | 10 (p.o.) | 10 | 55 | 45 |
| 9 | 30 (p.o.) | 10 | 70 | 120 |

All of the Compounds 1 to 7 were effective in suppressing decerebrate rigidity in rats, which is recognised as a model for spasticity and/or rigidity in humans. In particular among these, Compounds 1 and 2 proved to be more potent than eperisone hydrochloride (Compound 8) or as potent as afloqualone (Compound 9), both of which have already been used clinically as therapeutic agents for myotonia caused by cerebrovascular disorder sequelae. Accordingly, these two compounds were employed in Test 2.

Test 2: Suppression of decerebrate rigidity (cats)

Adult cats each weighing 3.0 to 4.5 kg were used. Under ether anesthesia, a tracheal cannula was applied, and the common carotid arteries were ligated at both sides. The cat was decerebrated by suctoin at the precollicular level. Ether anesthesia was then immediately discontinued. A pair of needle electrodes was inserted into the neck muscle in order to pick up any EMG muscular discharges. The EMG discharge was amplified and recorded on a pen-writing recorder, along with an integration of the EMG discharge. Recording was started at least 2 hours after withdrawal of anesthesia. Blood pressure induced from the femoral artery, as well as body temperature and heart beat, were recorded and monitored

throughout the experiment. After stable EMG discharges were obtained, the test compounds suspended in 0.5% CMC solution were administered into the duodenem through a previously inserted cannula. The effect of the test compound was expressed as the ratio of the integrated EMG discharge after administration relative to the value before administration.

At a dose of 100 mg/kg, both the Compounds 1 and 2 began to depress the decerebrate rigidity within 2 to 3 minutes of intraduodenal administration. About 30% and about 65% inhibitions were observed 5 minutes and 1 hour after administration, respectively. These inhibitory effects lasted throughout the observation period of 2 hours. On the other hand, similar administration of 50 mg/kg of eperisone hydrochloride (Compound 8), the control drug, induced vomiting immediately after administration accompanied with abrupt changes in blood pressure and heart beat. Although decerebrate rigidity was inhibited by eperisone hydrochloride, it returned 30 minutes after administration. This short duration was also observed in Test 1.

Thus, Compounds 1 and 2 reduced the decerebrate rigidity in cats, which is a model of myotonia as cerebral apoplectic sequelae. Both compounds were weaker in side effects and of longer action than eperisone hydrochloride, which is clinically used at present as a therapeutic agent for such a kind of condition.

Test 3: Effect on spinal reflex in cats

Adult cats weighing 3.0 to 4.5 kg each were used. After induction of anesthesia with ether, the animal was anesthetized by intravanous injection of 50 mg/kg of α-chloralose. A tracheal cannula was applied, the animal was fixed on a stereotaxic apparatus, and the lumbosacral spinal cord was exposed by laminoectomy from $L_3$ to $S_1$. The ventral roots of the right side were cut from $L_6$ to $S_1$ as distal as possible and the cut ends were ligated with cotton thread previously immersed in Linger's solution. The exposed spinal cord was covered with warmed mineral oil and maintained at 37°C. The ventral root, either $L_7$ or $S_1$, was fixed on a pair of platinum wire electrodes. A pair of collar-type electrodes was implanted on the ipsilateral tibial nerve and the saphenous nerve, for stimulation. After completion of all these operations, the cat was immobilized by intravenous injection of 0.5 mg/kg of pancronium bromide and maintained by artificial respiration (40/minute) Recording was started at least 2 hours after withdrawal of ether anesthesia. A single square pulse (0.01 to 0.1 msecond pulse width, supramaximal intensity) was applied to the nerve at a frequency of 0.3 Hz from an electronic stimulator. The spinal monosynaptic reflex, polysynaptic reflex, and spino-bulbo-spinal reflex recorded from the ventral root were amplified, displayed on an oscilloscope, and the signals were averaged over ten signals using a signal processor. In a similar manner to the Test 2, the blood pressure, heart beat and body temperature of the animal were recorded and monitored. Compounds to be tested were also administered in a similar manner.

After administration of a dose of 100 mg/kg of Compound 1 or Compound 2, both the polysynaptic reflex and the spino-bulbo-spinal reflexes were remarkably inhibited, though the monosynaptic reflex was little affected. The onset of this effect was observed 5 minutes after administration, reaching its maximum after 30 minutes and continuing for about 2 hours. A similar effect was observed by similar administration of 100 mg/kg of chlorphenesin carbamate. Therefore, Compounds 1 and 2 were shown to be interneuron blockers, like chlorphenesin carbamate.

Test 4: Potentiation of thiopental anesthesia (mice)

In the test method, male adult mice of DDY strain each weighing 20 to 30 g were used as divided groups each made up of 5 to 7 mice. Test compounds suspended in 0.5% CMC solution were orally administered. After one hour, thiopental (30 mg/kg) was injected into the tail vein, and the time required for the mouse to recover the righting reflex was measured.

The results are summarized in the following table.

Effect on duration of thiopental anesthesia

| Compound | Dose (mg/kg) | Animal number | Anesthesia time (sec.) |
|---|---|---|---|
| 1 | 0 | 7 | 254.7 ± 19.5 |
| 1 | 50 | 8 | 288.5 ± 20.1 |
| 1 | 100 | 7 | 237.3 ± 25.5 |
| 2 | 0 | 10 | 275.3 ± 18.6 |
| 2 | 100 | 9 | 329.1 ± 81.2 |
| 8 | 0 | 10 | 206.0 ± 13.1 |
| 8 | 10 | 5 | 182.0 ± 9.8 |
| 8 | 30 | 5 | 192.0 ± 18.3 |
| 8 | 100 | 5 | 247.0 ± 34.9 |
| 9 | 0 | 10 | 206.0 ± 13.1 |
| 9 | 10 | 5 | 242.0 ± 10.0 |
| 9 | 30 | 5 | 4657.0 ± 636.6* |

* : $P < 0.001$

Compounds 1 and 2 did not significantly prolong the duration of thiopental-induced anesthesia in comparison with eperisone hydrochloride (Compound 8), which has already been used clinically as a centrally-acting muscle relaxant. On the other hand, afloqualone (Compound 9) which has also been used for this purpose, did significantly prolong the duration of anesthesia at an effective dose of 30 mg/kg. This fact implies that Compounds 1 and 2 induce drowsiness (the adverse effect) less than afloqualone.

Test 5: Acute toxicity

Compound 1 was suspended in 0.5% CMC solution, and 1000 mg/kg of the compound was orally administered to each of 5 male adult mice of DDY strain (weighing 20 to 25 g each) which were then observed for 5 days. Systemic hypotonia attributed to the effect of the drug was observed for about 3 hours after administration, but all of the mice survived.

The same test was also performed, in turn, with Compound 2 and its hydrochloride, each administered to the mice at a dose of 500 mg/kg, in the same manner as before. Systemic hypotonia, attributed to the effect of the drug, was observed for about 3 hours after administration, but all of the mice survived over a period of 5 days.

Examples of Pharmaceutical Compositions

The present invention is further illustrated by the following examples.

Example 1: Tablets

120 mg tablets were prepared by conventional tabletting procedures based on the following formulation:

| | |
|---|---|
| 2-(2-Hydroxy-3-morpholinopropyl) -5-phenyl-4-isoxazolin-3-one | 10.0 mg |
| Corn starch | 25.0 mg |
| Lactose | 83.3 mg |
| HPC (Product of Nippon Soda., Ltd.) | 1.2 mg |
| Magnesium stearate | 0.5 mg |
| Total | 120.0 mg |

Example 2: Capsules

A powder was prepared in accordance with the following formulation, mixed well, and passed through a 60-mesh sieve. 280 mg aliquots of the sieved powder were put into gelatine capsules (No. 3) to give the filled capsules.

| | | |
|---|---|---|
| 2-(2-Hydroxy-3-morpholinopropyl) -5-phenyl-4-isoxazolin-3-one | | 25.0 mg |
| Lactose | | 153.6 mg |
| Corn starch | | 100.0 mg |
| Magnesium stearate | | 1.4 mg |
| | Total | 280.0 mg |

Example 3: Capsules

A powder was prepared in accordance with the following formulation, mixed well, and passed through a 60-mesh sieve. 280 mg aliquots of the sieved powder were put into gelatine capsules (No. 3) to give the filled capsules.

| | |
|---|---|
| 4-Chloro-(2-hydroxy-3-morpholino-propyl)-5-phenyl-4-isoxazolin-3-one | 25.0 mg |
| Lactose | 153.6 mg |
| Corn starch | 100.0 mg |
| Magnesium stearate | 1.4 mg |
| Total | 280.0 mg |

**Claims**

1. A process for the preparation of a pharmaceutical composition with centrally-acting muscle relaxant activity, characterized by the use as active compound of a compound of the formula:

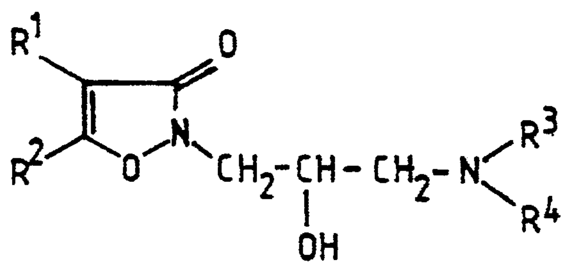

(I)

wherein:
$R^1$ is a hydrogen or halogen atom;
$R^2$ is an alkyl group with 1 to 4 carbon atoms, a phenyl group, a phenyl group substituted with 1 to 3 substituents, a heterocyclic group, or a heterocyclic group substituted with 1 to 3 substituents, the 1 to 3 substituents being selected from alkyl groups with 1 to 4 carbon atoms, alkoxy groups with 1 to 4 carbon atoms, hydroxy groups, halogen atoms, nitro groups, and/or trifluoromethyl groups, and the heterocyclic groups having 5 or 6 ring atoms which include 1 to 3 heteroatoms selected from oxygen, sulphur, and/or nitrogen heteroatoms; or
$R^1$ and $R^2$ together with the intervening carbon atoms form a 6- or 7-membered hydrocarbon ring;
$R^3$ is a hydrogen atom or an alkyl group having from 1 to 4 carbon atoms;
$R^4$ is an alkyl group having 1 to 4 carbon atoms; or

$R^3$ and $R^4$ together with the intervening nitrogen atom form a 5- or 6-membered alicyclic amino group optionally having at least one further heteroatom selected from oxygen, sulphur and/or nitrogen heteroatoms, the nitrogen heteroatom optionally being substituted with an alkyl group having 1 to 4 carbon atoms;
or a pharmaceutically acceptable salt thereof.

2. A process according to claim 1, wherein, in the active compound,
$R^1$ is as defined and $R^2$ is an alkyl group with 1 to 4 carbon atoms, a phenyl group, or a phenyl group substituted with 1 to 3 substituents, or
$R^1$ and $R^2$ together with the intervening carbon atoms form a benzene ring:
$R^3$ is a hydrogen atom and $R^4$ is an alkyl group having 1 to 4 carbon atoms; or
$R^3$ and $R^4$ together with the intervening nitrogen atom form an alicyclic amino group which is a 5-membered alicyclic amino group, or a 6-membered alicyclic amino group having at least one further heteroatom selected from oxygen heteroatoms, nitrogen heteroatoms and/or nitrogen heteroatoms substituted with an alkyl group having 1 to 4 carbon atoms.

3. A process according to claim 1, wherein, in the active compound,
$R^1$ is a hydrogen, fluorine, chlorine or bromine atom;
$R^2$ is an alkyl group with 1 to 4 carbon atoms, a phenyl group, or a phenyl group substituted with 1 to 3 substituents selected from fluorine atoms, chlorine atoms, bromine atoms, alkoxy group having from 1 to 4 carbon atoms, and/or hydroxy groups;
$R^3$ is a hydrogen atom and $R^4$ is an alkyl group having 1 to 4 carbon atoms; or
$R^3$ and $R^4$ together with the intervening nitrogen atom form a morpholino, 1-piperazinyl, 4-methyl-1-piperazinyl, or piperidino group.

4. A process according to claim 1, wherein the active compound is selected from
2-(2-hydroxy-3-morpholinopropyl)-5-phenyl-4-isoxazolin-3-one;
4-chloro-2-(2-hydroxy-3-morpholinopropyl)-5-phenyl-4-isoxazolin-3-one;
2-(2-hydroxy-3-morpholinopropyl)-5-methyl-4-isoxazolin-3-one;
5-P-chlorophenyl-2-(2-hydroxy-3-morpholinopropyl)-4-isoxazolin-3-one;
4-chloro-2-(2-hydroxy-3-isopropylaminopropyl)-5-phenyl-4-isoxazolin-3-one;
2-(2-hydroxy-3-isopropylaminopropyl)-5-phenyl-4-isoxazolin-3-one;
5-p-chlorophenyl-2-(2-hydroxy-3-isopropylaminopropyl)-4-isoxazolin-3-one; and/or
pharmaceutically acceptable salts of these compounds.

5. A process according to claim 4, wherein the active compound is selected from the group consisting of:
2-(2-hydroxy-3-morpholinopropyl)-5-phenyl-4-isoxazolin-3-one;
4-chloro-2-(2-hydroxy-3-morpholinopropyl)-5-phenyl-4-isoxazolin-3-one; and/or
pharmaceutically acceptable salts of these compounds.

6. A process according to any preceding claim, wherein the active compound is formulated for administration by an administration mode selected from oral administration and parenteral administration.

7. A process according to claim 6, wherein the active compound is formulated for admistration as a composition selected from tablets, capsules, granules, powders, syrups, injections or suppositories.

8. A process according to claim 6 or 7, wherein the active compound is formulated for administration at a dose of 5 mg to 50 mg given orally to adult humans, 1 to 3 times a day.

**Revendications**

1. Procédé pour la préparation d'une composition pharmaceutique ayant une activité myorelaxante par action centrale, caractérisée par l'utilisation d'un composé ayant la formule:
x

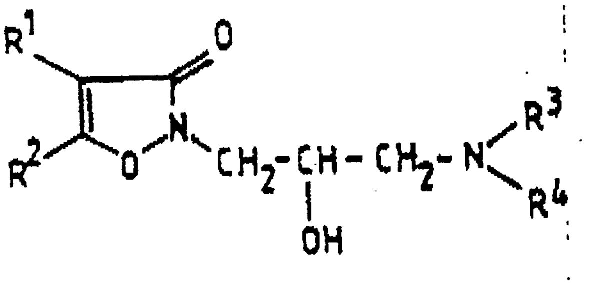

(I)

dans laquelle
$R^1$ est un atome d'hydrogène ou un atome d'halogène
$R^2$ est un groupe alkyle en C1–C4, un groupe phényle, un groupe phényle substitué par 1 à 3 substitants,

un groupe hétérocyclique ou un groupe hétérocyclique substitué par 1 à 3 substituants, lesquels 1 à 3 substituants étant choisis parmi des groupes alkyle en C1–C4, des groupes alkoxy en C1–C4, des groupes hydroxy, des atomes d'halogène, des groupes nitro, et/ou des groupes trifluorométhyle, et les groupes hétérocycliques ayant 5 ou 6 atomes dans le cycle qui comprennent 1 à 3 hétéroatomes choisis parmi les hétéroatomes oxygène, soufre, et/ou azote; ou

$R^1$ et $R^2$ forment conjointement avec les atomes de carbone intermédiaires un cycle hydrocarboné hexa- ou heptagonal;

$R^3$ est un atome d'hydrogéne ou un groupe alkyle en C1–C4;

$R^4$ est un groupe alkyle en C1–C4; ou

$R^3$ et $R^4$ forment conjointement avec l'atome d'azote intermédiaire un groupe amino alicyclique ayant facultativement au moins un hétéroatome supplémentaire choisi parmi les hétéroatomes oxygène, soufre et/ou azote, l'hétéroatome azote étant facultativement substitué par un groupe alkyle en C1–C4;

ou un sel pharmaceutiquement acceptable de ces composés.

2. Procédé selon la revendication 1, dans lequel, dans le composé actif,

$R^1$ est comme défini ci-dessus et $R^2$ est un groupe alkyle en C1–C4, un groupe phényle, ou un groupe phényle substitué par 1 à 3 substituants; ou

$R^1$ et $R^2$ forment conjointement avec les atomes de carbone intermédiaires un cycle benzène;

$R^3$ est un atome d'hydrogène et $R^4$ est un groupe alkyle en C1–C4; ou

$R^3$ et $R^4$ forment conjointement avec l'atome d'azote intermédiaire un groupe amino alicyclique qui est un groupe amino alicyclique pentagonal, ou un groupe amino-alicyclique hexagonal ayant au moins un hétéroatome supplémentaire choisi parmi les hétéroatomes oxygène, les hétéroatomes azote et/ou les hétéroatomes azote substitués par un groupe alkyle en C1–C4.

3. Procédé selon la revendication 1, dans lequel, dans le composé actif,

$R^1$ est un atome d'hydrogène, de fluor, de chlore ou de brome;

$R^2$ est un groupe alkyle en C1–C4, un groupe phényle, un groupe phényle substitué par 1 à 3 substitants choisis parmi les atomes de fluor, les atomes de chlore, les atomes de brome, un groupe alkoxy en C1–C4, et/ou des groupes hydroxy;

$R^3$ est un atome d'hydrogène et $R^4$ est un groupe alkyle en C1–C4; ou

$R^3$ et $R^4$ forment conjointement avec l'atome d'hydrogène intermédiaire un groupe morpholino, 1-pipérazinyle, 4-méthyl-1-pipérazinyle, ou pipéridino.

4. Procédé selon la revendication 1 dans lequel le composé actif est choisi parmi

le 2-(2-hydroxy-3-morpholinopropyl)-5-phényl-4-isoxyzoline-3-one;

le 4-chloro-2-(2-hydroxy-3-morpholinopropyl)-4-phényl-4-isoxazoline-3-one;

le 2-(2-hydroxy-3-morpholinopropyl)-5-méthyl-4-isoxazoline-3-one;

le 5-p-chlorophényl-2-(2-hydroxy-3-morpholinopropyl)-5-phényl-4-isoxazoline-3-one;

le 4-chloro-2-(2-hydroxy-3-isopropylaminopropyl)-5-phényl-4-isoxazoline-3-one;

le 2-(2-hydroxy-3-isopropylaminopropyl)-5-phényl-4-isoxazoline-3-one;

le 5-p-chlorophényl-2-(2-hydoxy-3-isopropylaminopropyl)-4-isoxazoline-3-one;

et/ou les sels pharmaceutiquement acceptables de ces composés.

5. Procédé selon la revendication 4, dans lequel le composé actif est choisi dans le groupe composé:

du 2-(2-hydroxy-3-morpholinopropyl)-5-phényl-4-isoxazoline-3-one;

du 4-chloro-2-(2-hydroxy-3-morpholinopropyl)-5-phényl-4-isoxazoline-3-one; et/ou des sels pharmaceutiquement acceptables de ces composés.

6. Procédé selon l'une quelconque des revendications précédentes dans lequel le composé actif est formulé pour l'administration par un mode d'administration choisi parmi les modes d'administration orale et les modes d'administration parentéraux.

7. Procédé selon la revendication 6, dans lequel le composé actif est formulé pour l'administration sous forme d'une composition choisie parmi les comprimés, les capsules, les granules, les poudres, les sirops, les injections et les suppositoires.

8. Procédé selon les revendications 6 et 7 dans lequel le composé actif est formulé pour un administration à une dose de 5 mg à 50 mg, donnée par voie orale à des adultes 1 à 3 fois par jour.

**Patentansprüche**

1. Verfahren zum Herstellen einer pharmazeutischen Mischung mit zentralwirkender Wirkung als Muskelrelaxans gekennzeichnet durch die Verwendung einer Verbindung, der Formel:

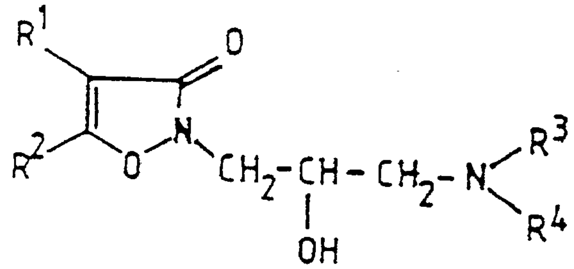

(I)

als Wirkstoff,
in welcher
$R^1$ ein Wasserstoffatom oder ein Halogenatom bedeutet,
$R^2$ eine Alkylgruppe mit 1 bis 4 Kohlenstoffatomen, eine Phenylgruppe, eine durch 1 bis 3 Substituenten substituierte Phenylgruppe, eine heterocyclische Gruppe oder eine durch 1 bis 3 Substituenten substituierte heterocyclische Gruppe darstellt, wobei die 1 bis 3 Substituenten aus Alkylgruppen mit 1 bis 4 Kohlenstoffatomen, Alkoxygruppen mit 1 bis 4 Kohlenstoffatomen, Hydroxygruppen, Halogenatomen, Nitrogruppen und/oder Trifluormethylgruppen ausgewählt sind und die 5 oder 6 Ringatome aufweisenden heterocyclischen Gruppen 1 bis 3 von Sauerstoff, Schwefel und/oder Stickstoff gebildete Heteroatome aufweisen, oder
$R^1$ und $R^2$ zusammen mit den dazwischenliegenden Kohlenstoffatomen einen 6- oder 7-gliedrigen Kohlenwasserstoffring bilden,
$R^3$ ein Wasserstoffatom oder eine Alkylgruppe mit 1 bis 4 Kohlenstoffatomen darstellt,
$R^4$ eine Alkylgruppe mit 3 bis 4 Kohlenstoffatomen bedeutet oder
$R^3$ und $R^4$ zusammen it dem dazwischen liegenden Stickstoffatom eine 5- oder 6-gliedrige alicyclische Aminogruppe bilden, welche gegebenenfalls zumindest ein weiteres aus Sauerstoff-, Schwefel- und/oder Stickstoffatomen gebildetes Heteroatom aufweist, wobei das Stickstoffheteroatom gegebenenfalls durch eine Alkylgruppe mit 3 bis 4 Kohlenstoffatomen substituiert ist,
oder eines pharmazeutisch annehmbaren Salzes hievon.

2. Verfahren nach Anspruch 1, worin im Wirkstoff
$R^1$ angegebene Bedeutung besitzt und $R^2$ eine Alkylgruppe mit 3 bis 4 Kohlenstoffatomen, eine Phenylgruppe oder eine durch 3 bis 3 Substituenten substituierte Phenylgruppe bedeutet oder
$R1$und $R2$ zusammen mit den dazwischenliegenden Kohlenstoffatomen einen Benzolring bilden,
$R3$ ein Wasserstoffatom bedeutet und $R^4$ eine Alkylgruppe mit 1 bis 4 Kohlenstoffatomen darstellt oder
$R^3$ und $R^4$ zusammen mit dem dazwischenliegenden Stickstoffatom eine alicyclische Aminogruppe bilden, welche eine 5-gliedrige alicyclische Aminogruppe ist oder eine 6-gliedrige alicyclische Aminogruppe darstellt, welche zumindest ein weiteres Heteroatom aufweist, das aus Sauerstoffheteroatomen, Stickstoffheteroatomen und/oder durch eine Alkylgruppe mit 1 bis 4 Kohlenstoffatomen substituierten Stickstoffheteroatomen ausgewählt ist.

3. Verfahren nach Anspruch 1, worin im Wirkstoff
$R^1$ ein Wasserstoffatom oder ein Fluor-, Chlor- oder Bromatom bedeutet,
$R^2$ eine Alkylgruppe mit 3 bis 4 Kohlenstoffatomen, eine Phenylgruppe oder eine Phenylgruppe darstellt, welche durch 1 bis 3 aus Fluoratomen, Chloratomen, Bromatomen, Alkoxygruppen mit 1 bis 4 Kohlenstoffatomen und/oder Hydroxygruppen ausgewählten Substituenten substituiert ist,
$R^3$ ein Wasserstoffatom bedeutet und $R^4$ eine Alkylgruppe mit 3 bis 4 Kohlenstoffatomen darstellt oder
$R^3$ und $R^4$ zusammen mit dem dazwischenliegenden Stickstoffatom eine Morpholino-, 1-Piperazinyl-, 4-Methyl-1-piperazinyl- oder Piperidinogruppe bilden.

4. Verfahren nach Anspruch 1, worin der Wirkstoff aus 2-(2-Hydroxy-3-morpholinopropyl)-5-phenyl-4-isoxazolin-3-on;
4-Chlor-2-(2-hydroxy-3-morpholinopropyl)-5-phenyl-4-isoxazolin-3-on;
2-(2-Hydroxy-3-morpholinopropyl)-5-methyl-4-isoxazolin-3-on;
5-p-Chlorphenyl-2-(2-hydroxy-3-morpholinopropyl)-4-isoxazolin-3-on;
4-Chlor-2-(2-hydroxy-3-isopropylaminopropyl)-5-phenyl-4-isoxazolin-3-on;
2-(2-Hydroxy-3-isopropylaminopropyl)-5-phenyl-4-isoxazolin-3-on;
5-p-Chlorphenyl-2-(2-hydroxy-3-isopropylaminopropyl)-4-isoxazolin-3-on; und/oder pharmazeutisch annehmbaren Salzen dieser Verbindungen ausgewählt ist.

5. Verfahren nach Anspruch 4, worin der Wirkstoff aus der von 2-(2-Hydroxy-3-morpholinopropyl)-5-phenyl-4-isoxazolin-3-on;
4-Chlor-2-(2-hydroxy-3-morpholinopropyl)-5-phenyl-4-isoxazolin-3-on; und/oder pharmazeutisch annehmbaren Salzen dieser Verbindungen gebildeten Gruppe ausgewählt ist.

6. Verfahren nach irgendeinem vorhergehenden Anspruch, worin der Wirkstoff für die Verabreichung nach einem Verabreichungsweg formuliert ist, welcher aus oraler Verabreichung und parenteraler Verabreichung ausgewählt ist.

7. Verfahren nach Anspruch 6, worin der Wirkstoff für die Verabreichung in Form einer Mischung formuliert ist, die aus Tabletten, Kapseln, Granulat, Pulvern, Sirups, Injektionen oder Suppositorien ausgewählt ist.

8. Verfahren nach Anspruch 6 oder 7, worin der Wirkstoff für die Verabreichung in einer Dosis von 5 mg bis 50 mg formuliert ist, die an erwachsene Menschen oral 1- bis 3-mal je Tag verabreicht wird.